(19) Europäisches Patentamt
European Patent Office
Office européen des brevets

(11) Publication number: **0 028 525**
**A2**

(12) # EUROPEAN PATENT APPLICATION

(21) Application number: 80303900.7

(51) Int. Cl.$^3$: A 61 K 7/06

(22) Date of filing: 31.10.80

(30) Priority: 31.10.79 FI 793410

(43) Date of publication of application: 13.05.81 Bulletin 81/19

(84) Designated Contracting States: AT BE CH DE FR GB LI NL SE

(71) Applicant: Orion-yhtymä Oy
PL 19
00101 Helsinki 10(FI)

(72) Inventor: Hannuksela, Hannu Matti Jaakko
Kastellintie 77
SF-90230 Oulu 23(FI)

(72) Inventor: Keranen, Antti Jaakko
Kouvusyrja 10 B
SF-02180 Espoo 18(FI)

(72) Inventor: Sothmann, Gunnar Aslak
Kaksomaki 18
SF-92400 Kirkkonummi(FI)

(74) Representative: Warden, John C. et al,
R.G.C. Jenkins & Co. Chancery House 53/64 Chancery Lane
London WC2A 1QU(GB)

(54) **Hair treatment agent promoting the growth of hair and preventing dandruff.**

(57) A hair treatment composition for topical use to promote the growth of hair and prevents dandruff which contains spironolactone as the active component. The composition is obtained by mixing spironolactone with additives and auxiliary agents.

EP 0 028 525 A2

~~Orion-yhtymä Oy~~

Hair treatment agent promoting the growth of hair and preventing dandruff

The subject of the present invention is a hair treatment agent that promotes the growth of hair and prevents dandruff and that is characterized in that it contains spironolactone as the active component.

The basic factor of alopecia androgenetica (typical baldness of men and some women at the temples and on the top of the head) is obviously a hormonal one, for the trouble starts only after the age of puberty, when the production of sex hormones increases extensively. This phenomenon does not occur in eunuchs (J. Hamilton, Ann.J.Anat. 71, 451-80 /¯1942_7). In apes it is possible to induce baldness by means of testosterone (I. Takashima and W.Montagna, Arch.Dermatol. 103, 522-34 /¯1971_7). In the hair root, testosterone is converted into some other active androgenic hormones, the most important of which are $\Delta^4$-androstendione, 5$\alpha$-dihydrotestosterone, 5$\alpha$-androstendione, and androsterone (A.Fazekas and A.Lanthier, Steroids 18, 367-79 /¯1971_7). This conversion is clearly stronger within the area becoming bald than within an area in which hair is not being lost (K.Adachi and M.Kano, Biochem. Biophys.Res.Commun. 41, 884-90 /¯1970_7).

We know about the sebaceous glands that they include an abundance of androgen receptors. The same androgens as affect the hair roots also have a stimulating effect on the sebaceous glands. The consequence is Seborrhea oleosa and infected dandruff of the scalp (J.Strauss and P.Pochi, Dermatotoxicology and Pharmacology, edited by F.Marzulli and H.Maibach, Hemisphere Publishing Co., Washington, 231-45 /¯1977_7). By blocking the androgen receptors by means of anti-androgens, it is possible to reduce the production of sebum (H.Zaun and E.Ludwig, Z.Hautkr. 53, 759-65 /¯1978_7). These receptors have not been studied more extensively from the roots of different hairs. On the other hand, we know that the 5-reductase activity has been increased in areas becoming bald (H.Schweikert and J.Wilson, J.Clin.Endocrinol.Metab. 38, 811-9 /¯1974_7), which increases the quantity of 5α-dihydrotestosterone in these areas. On the other hand, this hormone inhibits the adenyl-cyclase system, which again results in inhibition of protein synthesis (K.Adachi, Curr.Probl.Dermatol. 5, 37-78 /¯1973_7). If the reduction in hair took place in accordance with this hypothesis, androgen receptors would not be necessarily needed in the hair roots. That would, however, leave the question unanswered, why other hairs (except those growing on top of the head) are stimulated by an addition of androgen.

Progesterone, which is in many respects a competitor of testosterone, increases the quantity of hairs at the growing stage in the scalp to a considerable extent (P.Mauvais-Jarvis et al., J.Clin.Endocrinol.Metab. 38, 142-7 /¯1974_7). Among antiandrogens, with this view, especially cyproterone acetate has been studied, and it has been noticed that it has a strong effect on the activity of both the sebaceous glands and the hair roots (F.Ebling et al., Brit.J.Dermatol. 97, 371-81 /¯1977_7). Attempts have also been made to use cyproterone topically, but so far it has not been possible to establish its effect on the sebaceous glands or on hair growth. This is probably because of poor penetration or too low concentrations at the affecting point.

The present invention is based on the surprising observation that spironolactone has a strong effect increasing the growth of hair in the scalp and preventing dandruff even when used topically. Besides being an antagonist to aldosterone, spironolactone is also an anti-androgen. It functions both as an agent inhibiting the production of testosterone (P.Corvol et al., Endocrinology 97, 52-8 /¯1974_7) and as an agent blocking testosterone receptors (J.Pita et al., Endocrinology 97, 1521-7 /¯1975_7).

Spironolactone has already been used for years for cardiac insufficiency together with diuretics, and no serious side effects have been indicated with the exception of patients suffering from difficult diseases of the liver or kidney (see Extra Pharmacopoeia Martindale 27, 567-9 /¯1977_7). Its toxic and teratogenic properties are well mapped-out.

The topical use of spironolactone in alopecia androgenetica and in different forms of seborrhea is based on its anti-androgenic properties. Owing to its steroid structure and to its solubility in lipids, it is capable of penetrating down into the hair roots to a sufficient extent. Topical usability is a highly remarkable advantage of spironolactone as compared with the steroids to be administered orally, e.g. cyproterone. The detrimental side effects, mainly feminisation, noticed in the latter compounds as well as in estrogens used topically have not been noticed in connection with the use of spironolactone. Spironolactone can be used for patients of all ages, both men and women, because the effect is directed at the area of application alone.

The effect of the hair treatment agent in accordance with the present invention has been established by means of a clinical study. For patients were selected persons who had strong or moderate alopecia androgenetica so that the temples had already become bald and the top of the head had also quite scarce hair if any left. All the patients had additionally seborrhea sicca (dandruff) or seb. oleosa (strong oiliness) or both of these.

As treatment was used 1-% spironolactone liniment, which was applied by the patients daily after washing 10 to 30 drops onto the area becoming bald. Controls were performed at intervals of 2 to 3 months, at which times both changes in hair growth and the loosening quality of the hairs were recorded. Moreover, the condition of the scalp was ascertained.

The results are given in the attached table. In the case of six patients, out of the total twelve, attempts had been made previously to treat the seborrhea by means of betamethasone liniment (a synthetic glucocortico-steroid that inhibits infection and that has no anti-androgenic effect), and in the case of two patients additionally by means of tetracycline (0.1 or 0.25 x 1) (an antibiotic whose effect is based on inhibition of the local growth of bacteria in the sebaceous glands and on making the sebum more fluid) during several months. The treatment usually had a favourable effect on seborrhea forms but no effect on alopecia.

The duration of the spironolactone treatment varied from 2 to 15 months. With four patients a very good result was obtained in alopecia androgenetica, and in addition to them a good result with three patients, i.e. a total of 7/12 patients reacted favourably to this treatment. Slight improvement was registered with one female patient, with whom the loss of hair was stopped. With the remaining four patients a good deal of new hair was indeed seen in particular in the area of the temples, but the hairs grew only to the length of 1 to 2 cm and became loose. On the basis of the results obtained, spironolactone treatment is more efficient than any earlier treatment used for alopecia androgenetica.

The spironolactone treatment also proved highly efficient in seborrhea. Seven patients became practically free from symptoms, and besides them the results were good with four patients. The result was questionable with one patient only.

Table

1-% Spironolactone liniment in alopecia androgenetica and in connected seborrhea

| Patient | Age | Sex | Alopecia androgenetica | Dandruff | Seborrhea (oiliness) | Duration | Previous treatment/ result | Spironolactone treatment mon. | Result/alopecia androg. | Result/ seborrhea |
|---|---|---|---|---|---|---|---|---|---|---|
| EK | 27 | M | xx | | xx | 10 y. | no treatment | 7 | +++/abundant new hair, did not become loose | ++ |
| SS | 31 | F | xxx | xxx | xxx | 8 y. | tetracycline 0.25xl 3 months betamethasone scalp/seborrhea ++, alopecia - | 6 | +++/could stop using peruke | + |
| IT | 22 | M | xx | xx | xx | 2 y. | no treatment | 11 | ++/abundant new hair at edge of scalp, did not become loose | +++ |
| PM | 27 | M | xx | xxx | xx | 8 y. | no treatment | 8 | -/some new hair, lost like before | +++ |
| TR | 35 | F | xx | xx | | 2 y. | betamethasone scalp/ good effect on dandruff | 4 | +/loss of hair stopped, no additional growth | ++ |

Table

1-% Spironolactone liniment in alopecia androgenetica and in connected seborrhea

| Patient | Age | Sex | Alopecia androgenetica | Dandruff | Seborrhea (oiliness) | Duration | Previous treatment/ result | Spironolactone treatment mon. | Result/alopecia androg. | Result/ seborrhea |
|---|---|---|---|---|---|---|---|---|---|---|
| AK | 28 | F | xx | | xxx | 6 y. | tetracycline 0.1 to 0.25x1 and betamethasone scalp/effect on seborrhea good | 14 | ++/on temples new hair moderately, did not become loose | +++ (no symptoms) |
| PK | 27 | M | xx | xx | xxx | 7 y. | betamethasone scalp: no help, tetracycline: for seborrhea ++ | 6 | ++/new hair at edge of scalp, did not become loose | ++/dandruff +++/seborrhea |
| EL | 40 | M | xx | x | xxx | 12 y. | no treatment | 11 | -/at first abundant new hair, was lost | +++ |
| KA | 38 | M | xx | x | xxx | 18 y. | betamethasone scalp/ dandruff was gone, no other help | 15 | +++/new hair about 50 % more, was not lost | +++ |
| LS | 32 | M | xx | xx | xxx | 15 y. | no treatment | 6 | -/abundant new stubble, was lost | +++ |
| EM | 25 | M | xx | xx | xx | 5 y. | betamethasone scalp/ dandruff was gone | 9 | +++/about 30 % new hair, was not lost | +++ |
| HH | 18 | M | xxx | x | xxx | 2 y. | no treatment | 15 | -/abundant new stubble, was lost | ++ |

(Explanations of the table)

Explanations:

| | | |
|---|---|---|
| x | = | slight symptoms |
| xx | = | medium symptoms |
| xxx | = | difficult symptoms |
| - | = | no effect or only little help |
| + | = | gave some help |
| ++ | = | was clearly helpful |
| +++ | = | gave very good help/ totally free from symptoms |

The age of the patient is given as years. The figure in the duration column means the duration of symptoms in years before spironolactone treatment.

The hair treatment agent in accordance with the invention is preferably used in solution form (as liniment), whereby the preparation can be formulated either as a mere solution, as a gel, dispersion, or emulsion depending on whether properties drying or oiling the skin are desired.

As solvents can be used volatile monohydric aliphatic alcohols, such as ethanol and propanol. Further favourable solvents are di- and polyhydric alcohols, such as glycerol, propylene glycol, and polyethylene glycol, because, besides functioning as solvents, they at the same time function as humectants of the skin, which is adapted to improve the absorption. A particularly favourable solvent is propylene glycol, which additionally also functions as a bactericidal agent.

If a preparation of higher drying property is desired, part of the poorly volatile solvent (e.g. of propylene glycol) may be substituted for by a more readily volatile one (e.g. 10 to 20 per cent of ethanol or propanol).

The quantity of the solvent of spironolactone is preferably selected so that it is as little below the saturation limit as possible. Then the distribution between the solvent and the skin can be optimized. An appropriate concentration of spironolactone is, e.g., 0.01 to 2.0 %. Most commonly a 1-% preparation was experimented with. At higher concentrations the quantity of alcohol or corresponding organic volatile solvent must be made higher, which again causes drying of the skin. With lower concentrations it is easier to obtain bases with different properties, but then the preparation must perhaps be used as larger quantities or more frequently, which is a factor disturbing the treatment.

By using various known auxiliary agents, it is possible to control the technical properties of the preparation and to make the preparation meet the requirements of the skin at each particular time to be treated.

An increase in viscosity in view of facilitating the administration can be achieved by including some cellulose soluble in water in the formulation, e.g. Na-carboxymethylcellulose, as well as water. It is also possible to use cellulose types that form gels without water, such as ethylcellulose or hydroxypropylcellulose. For very dry skin it is possible to formulate a preparation which, besides solvents, contains some fat soluble in the solvents. Alternatively, it is possible to do so that the fat component (vaseline, paraffin, wool-grease alcohols, or any other substance commonly used as a skin treatment agent) is emulsified into the base by using generally known ionic or non-ionizable emulsifiers by means of known technological processes.

Below some examples are given on formulations of hair treatment agents in accordance with the present invention, to which examples the invention is, however, not restricted. The figures in the examples denoting substance quantities are units of weight.

Example 1

| | |
|---|---|
| Spironolactone | 1.0 |
| Propylene glycol | 10.0 |
| Polyethylene glycol 400 (Macrogol 400) | 89.0 |

The spironolactone is dissolved into a mixture of propylene glycol and polyethylene glycol at about 40 to 60°C.

Example 2

| | |
|---|---|
| Spironolactone | 1.0 |
| Na-carboxymethylcellulose | 1 to 4 |
| Spiritus fortis | 10 to 50 |
| Propylene glycol + macrogol 400 | 10 to 40 |
| Aq. purif. | ad 100 |

A homogeneous slime is prepared out of Na-carboxymethylcellulose and water. The spironolactone is mixed into the mixture of propylene glycol and macrogol as indicated above. The solutions are combined with each other.

Example 3

| | |
|---|---|
| Spironolactone | 1.0 |
| Spiritus fortis | 20.0 |
| Propylene glycol | 10.0 |
| Macrogol 400 | 68.8 |
| Hydroxypropylcellulose | 0.2 |

The hydroxypropylcellulose is dissolved into the spirit to make a uniform slime. The spironolactone is dissolved as above, and the obtained solutions are combined.

Example 4

| | |
|---|---|
| Spironolactone | 1.0 |
| Spiritus fortis | 20 to 40 |
| Propylene glycol | 5 to 20 |
| Vinylpyrrolidone-vinylacetate-copolymer (Luviskol VA 64 R) | q.s. |
| Castor oil or Cremophor RH 40 R | q.s. |
| Aq. purif. | ad 100 |

The castor oil or equivalent is dissolved into the spirit. The propylene glycol is added, and the spironolactone is dissolved into this mixture under heating. Luviskol is dissolved into water. The solutions are combined.

0028525

CLAIMS:

1) A hair treatment composition for topical use to promote the growth of hair and prevent dandruff, characterised in that it contains spironolactone as an active ingredient.

2) A hair treatment composition according to Claim 1 characterised in that it also contains a glycol.

3) A hair treatment composition according to Claim 2 characterised in that said glycol is propylene glycol.

4) A hair treatment composition according to Claim 2 or Claim 3 characterised in that it contains polyethylene glycol.

5) A hair treatment composition according to any preceding Claim characterised in that it also contains spiritus fortis.